# EUROPEAN PATENT APPLICATION

(11) **EP 1 873 235 A1**
(43) Date of publication of application: **02.01.2008**
(21) Application number: 06425435.2
(22) Date of filing: 26.06.2006
(51) Int. Cl.: C12N 3/00, C12N 1/14, A23C 19/00

(54) **A method for the preparation of Penicillium spores and the use of the latter in the food field**

(71) Applicant: Universita' degli Studi di Milano, 20122 Milano (IT)
(72) Inventor: Mora, Diego, 20133 Milano (IT); Pintus, Paola, 20020 Arconate (Milano) (IT)
(74) Representative: Long, Giorgio

(57) **Abstract**

The present invention relates to a method for the preparation of *Penicillium* spores, and the use of the latter in the food field. Particularly, the invention relates to a method for the preparation of *Penicillium roqueforti* spores from a solid substrate and the use of the same in the preparation of marbled cheeses such as Roquefort, Gorgonzola, Stilton and Blue Cheese.

## Description

### TECHNICAL FIELD OF THE INVENTION

The object of the present invention is a method for the preparation of *Penicillium* spores, and the use of the latter in the food field. Particularly, the invention relates to a method for the preparation of *Penicillium roqueforti* spores from a solid substrate and the use of the same in the preparation of marbled cheeses such as Roquefort, Gorgonzola, Stilton and Blue Cheese.

### BACKGROUND OF THE INVENTION

It is known that the fungi belonging to the genus *Penicillium* are widely used in the food field, and particularly in the diary sector, to produce, for example, cheeses with particular organoleptic characteristics.

For example, worthy of mention among the various *Penicillium* genuses are the species *Penicillium candidum,* normally used in the production of cheeses such as Camembert and Brie, and *Penicillium roqueforti* commonly used to make the aforementioned cheeses such as Roquefort, Gorgonzola, Stilton and Blue Cheese.

In particular, the species *Penicillium roqueforti* is a fungus historically known to form and grow on products such as bread. At the industrial level, its production involves bringing fungal spores into contact with wheat or barley flour based substrates. Said substrate allows the fungus to reproduce under optimum conditions.

Once the desired quantity of fungus is obtained, this is harvested and used to conventionally inoculate dairy products to obtain cheeses with organoleptic characteristics typical of the marbled cheeses. Indeed, normally, preparations of such microorganisms are added to milk in the cheese production vat, followed by all the usual production stages.

It has been observed that suspensions of spores of this microorganism may contain, and release into the finished product, variable quantities of gluten (tens of ppm), a protein found typically in many cereals, including wheat, with which the microorganism's growth substrate (bread) is produced.

Consequently, consumption of foodstuffs containing even small amounts of gluten is strongly discouraged for individuals suffering from intolerance to this protein (coeliac disease).

### SUMMARY OF THE INVENTION

Therefore, the technical problem underlying the present invention is that of providing a dairy food product comprising *Penicillium roqueforti* that can also be consumed by those individuals affected by coeliac disease, without risking the serious problems of gluten intolerance.

Said problem is resolved by a method which allows provision of gluten-free *Penicillium roqueforti.*

Therefore, a first object of the invention is that of providing a method for the preparation of *Penicillium roqueforti* spores that are gluten-free.

A second object, is that of providing a process for producing a food product comprising *Penicillium roqueforti* that obtainable according to the aforementioned method as well as dairy products that can be obtained by means of said process.

### BRIEF DESCRIPTION OF THE FIGURES

Further characteristics and advantages of the present invention will become more evident from the following description of one embodiment, given purely by way of non-limiting example, with reference to the enclosed figures, in which:
- figure 1 represents a graph of the effect of citric acid concentration on sporulation of *Penicillium roqueforti* MIMZOL1 growing on husked, white rice grains;
- figure 2A shows a graph of the sporulation curve of Penicillium roqueforti MIMZOL1 on husked, white rice grains and on wholemeal rice grains;
- figure 2B shows a graph of the sporulation curve of Penicillium roqueforti MIMDAN1 on husked, white rice grains and on wholemeal rice grains;
- figure 3 represents a graph of the sporulation curves of Penicillium roqueforti MIMZOL1, MIMDAN1 and MIMZOL2 on husked, white rice grains.

### DETAILED DESCRIPTION OF THE INVENTION

The idea at the heart of the present invention is that of devising a method for producing the microorganism of interest in such a way that it is completely gluten-free.

Studies aimed at obtaining such a method have lead to the selection of culture media or substrates different to the conventional substrates containing gluten, such as conventional bread.

In particular, it has been surprisingly found that if *Penicillium roqueforti* is grown on a starchy substrate such as rice or potato, the microorganism is capable of growing and multiplying without any complications, such as to obtain a biomass of spores suitable for inoculating food products.

Indeed, it has been observed for example, that a growth substrate such as rice allowed complete growth in relation to the nutritional requirements of *Penicillium* without requiring the significant implication of nitrogen, carbon and vitamin sources.

Thus, the method for the preparation of gluten-free *Penicillium* spores, comprises a stage of bringing *Penicillium* spores into contact with a starchy substrate and a stage of maintaining said spores on said substrate for a suitable amount of time until obtaining the desired growth of gluten-free *Penicillium.*

The microorganism used belongs to the genus *Penicillium,* preferably strains of the species *Penicillium roqueforti.*

The method can include a preliminary stage of the preparation and maintenance of *Penicillium* strains. Said stage comprises the collection of *Penicillium* strains from whatever conventional source and bringing them into contact with a suitable growth medium. For example, a growth medium such as bread, or a medium containing starches, simple carbohydrates and agar, such as PDA (Potato Dextrose Agar), preferably rice, may be used.

The microorganisms are maintained at 30 °C for 3-5 days. Once the desired level of sporulation (10⁷-10⁸ spores/ml) has been obtained, the spore suspension may be stored in accordance with conventional procedures (at 4 °C or - 20 °C in acidic saline solutions, with or without glycerol). Preferably, if the aforementioned PDA medium, or the like, is used, the suspension is stored at a temperature ranging between -20 and -80°C in isotonic saline (9 g/l NaCl) or, more preferably, in any solution containing an organic acid as a carbon source and/or capable of solubilising and providing the metals normally used by microorganisms for performing their metabolic activities (Fe⁺², Mg⁺² and the like).

Furthermore, the solution may be supplemented with a cryoprotective substance, such as glycerol.

Preferably, the solution contains citrate buffer, supplemented with glycerol at a final concentration of 15% (v/v). More preferably, the buffer is 0.13 M citrate buffer, pH 4.5, and the glycerol is present at a final concentration of 15% v/v.

Once the microorganism is prepared in the above-described manner, the method comprises a stage of pre-inoculation on gluten-free medium, such as that described above, preferably PDA.

In particular, one or more strains of *Penicillium* are maintained on PDA for a length of time ranging between 3-5 days, under aerobic conditions, and at a temperature ranging between 18°-30°C in order to allow growth and sporulation of the microorganism, such as to obtain the above-specified quantities.

After said pre-inoculation, the biomass obtained is resuspended in a solution containing an organic acid, such as citrate buffer, such as to obtain a concentration ranging between 10⁶ and 10⁸ spores/ml, and used to inoculate the definitive growth substrate. As already mentioned, said substrate may be generally represented by any gluten-free starchy substrate.

Preferred substrates for growing *Penicillium* include potatoes and rice, more preferably, rice.

Particularly, the rice may be any kind of commercially available rice, such as husked, white rice or wholemeal rice, sterilised prior to inoculation with *Penicillium.* Preferably, the rice is husked, white rice subjected to two cycles of sterilisation at a temperature ranging between 111 °C and 116 °C for a period of time ranging between 20 and 30 minutes.

Inoculation occurs by the simple addition of a quantity of solution, prepared as described above, containing the spores at a concentration ranging between 10⁶ and 10⁷ spores/ml directly into containers containing a predetermined amount of rice (50 g - 2 kg) such as to give a loading of spores/g of rice ranging between 10⁵ and 10⁶.

The method then consists of an aerobic incubation stage at a temperature ranging between 15°C and 30°C for 4-15 days, preferably at 28°C for around 4 to 7 days, such as to achieve a sporulation level ranging between 10⁶ spores/g and 10⁹ spores/g of rice, preferably 10⁷ spores/g of rice.

During incubation, the containers are positioned such as to allow maximum aeration of the spores, and continually agitated at regular intervals such as to allow homogeneous aeration, even at the most concealed areas of growth substrate.

Once the incubation period is completed, the sporulation stage is interrupted, for example, by means of the addition of an appropriate volume (100 ml - 3 1) of an acidic solution for every 50 g - 2 kg of rice, preferably in a variable ratio of from 1:1 to 1:1.5. Preferably, the acidic solution is composed of a saline solution supplemented with lactic acid, and sterilised.

The method of the invention may further comprise a stage involving the separation of the spores from the culture medium. For example, the separation stage may occur by means of filtration.

In particular, filtration may be performed using a Buckner funnel fitted with a filter with a cut-off of 1 mm.

The spore suspension, obtained in accordance with the method described above, may be used to inoculate a food base, for example a dairy product, such as to produce, for example, marbled cheeses.

The amount of spores which can be used in cheese production can vary depending on the type of cheese desired, in accordance with entirely conventional production processes. In any case, it is preferable to inoculate 1000-2000 1 of milk with amounts varying from 10⁸ to 10⁹ spores, more preferably approx. 3×10⁹ spores or 1-2×10¹⁰ for each 5000-10000 1 of milk.

From the previous description, the method for the preparation of *Penicillium* spores, and the use of the latter in the food field, advantageously allows the provision of spores that are completely free of the protein gluten, the cause of coeliac disease.

Consequently, the method allows the use of *Penicillium* spores to produce foodstuffs, such as cheeses, that are entirely safe for those affected by the aforementioned disease.

It should also be noted that the method of the invention advantageously allows such significantly accelerated growth of *Penicillium,* that the entire production process of the product may be speeded up.

Furthermore, use of a substrate such as grains of rice allows rapid and efficient separation of the fungal spores from the biomass and the spent substrate (rice). Hence the described method allows the attainment of purer spore suspensions, if compared to those obtained using the traditional system, where the spores, spent substrate (bread) and fungal biomass are mixed together in an individual final suspension.

In particular, it has been observed that use of an organic acid, particularly citrate buffer, in the preparation of the spore suspension to be used as an inoculum for the solid rice substrate, has been surprisingly efficient for obtaining a high level of growth and sporulation for the fungal mycelium, and extremely short production times.

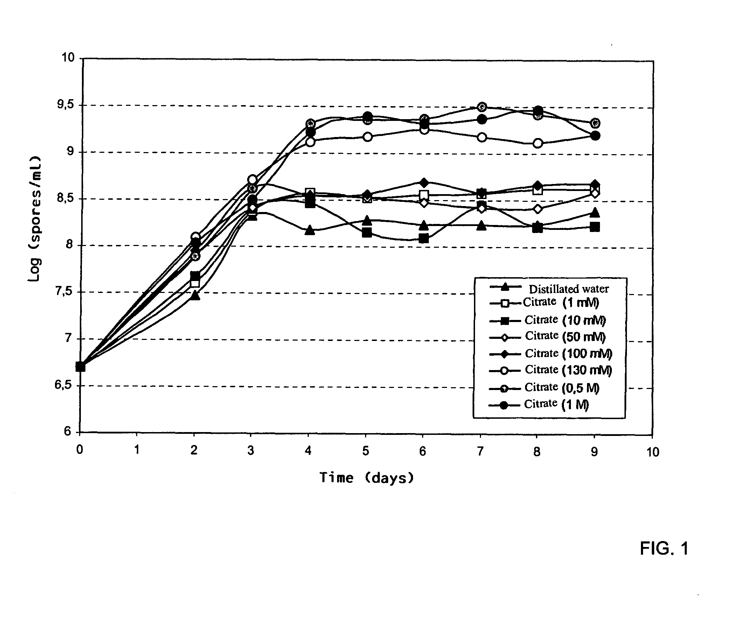

In figure 1, the *Penicillium* spore concentration is indicated as the log₁₀ of the number of spores per ml, measured by light microscope using a Petroff-Hausser counting chamber. The concentrations of citrate buffer present in the spore suspension used as an inoculum for the rice substrate, are indicated in the panel next to the graph. The incubation has been conducted at 28 °C.

Particularly, it has been observed that the appearance of the rice becomes markedly darker when organic acid concentrations between 130 mM and 1 M are used. In other words, under such conditions, the spores are produced faster, and completely cover the rice, while spore growth is slower at concentrations between 1 mM and 100 mM. Consequently, the organic acid is used at a concentration ranging between 1 mM and 1 M, preferably between 130 mM and 1 M.

Thanks to the introduction of an organic acid, such as citrate buffer, in the sterile rice grain inoculation stage, the process described allows significant reductions in production time from the 15-20 days, traditionally used in the traditional production systems using bread, to around 4-6 days. This reduction in production time to just a few days of incubation will allow manufacturers to make considerable savings in production costs, with easier management of orders.

The use of organic acid based buffers in concentrations varying between 130 mM and 1 M in the rice inoculation stage, is therefore to be considered a particularly preferred step in the production process underlying this invention.

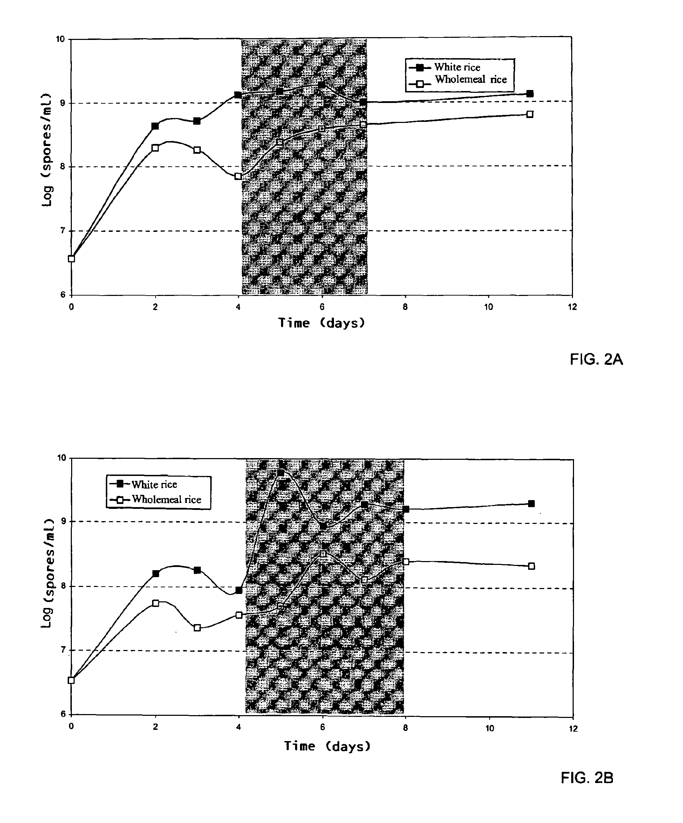

Figures 2A and 2B show the sporulation curves for certain strains of *Penicillium roqueforti (MIMZOL1, MIMDAN1: DISTAM microorganism collection - Industrial Microbiology section, Milan - Italy),* isolated from marbled cheeses, using both husked, white rice grains and wholemeal rice grains as substrate. The *Penicillium* spore concentration is indicated as the log₁₀ of the number of spores per ml measured by light microscopy using a Petroff-Hausser counting chamber. The shaded area shows the incubation stage, where maximum sporulation levels have been achieved. The incubation has been conducted at 28 °C.

As may be observed form the experimental data, both substrates used allow the attainment of high sporulation levels in very short times (10⁸-10⁹ spores/ml in 5-7 days of incubation). However, it should be observed that husked, white rice allows significantly higher *Penicillium* sporulation levels, with respect to wholemeal rice (Fig. 2A). Therefore, this substrate should be considered the most preferred.

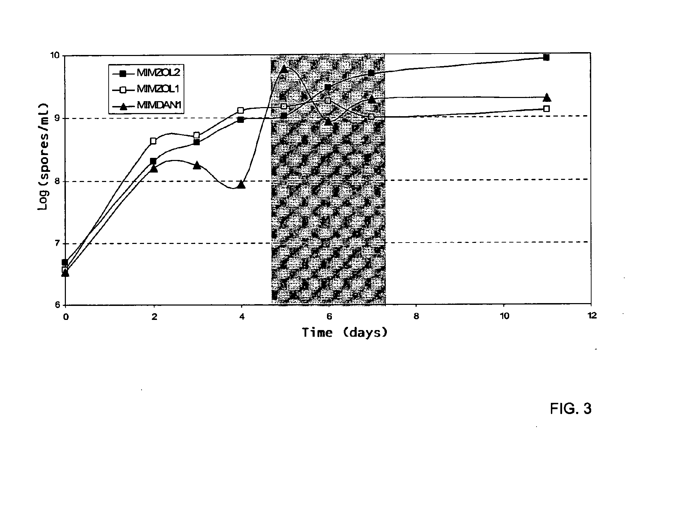

By way of example, Figure 3 shows the sporulation curves of three different strains of *Penicillium roqueforti* (*MIMZOL1*, *MIMDAN1, MIMZOL2: DISTAM microorganism collection* - *Industrial microbiology section, Milan - Italy)* and the photographs describing the relevant steps in the growth of the microorganism on the culture substrate, white rice grains. The *Penicillium* spore concentration is indicated as the log₁₀ of the number of spores per ml measured by light microscopy using a Petroff-Hausser counting chamber. The shaded area shows the incubation stage, where maximum sporulation levels have been achieved. The incubation has been conducted at 28 °C.

A non-limiting example of the production of *Penicillium* spores, and an example of gorgonzola production using the spores thus obtained is reported hereinafter.

### EXAMPLE 1

### Maintaining strains of Penicillium roquefortii

The *Penicillium roquefortii* strains used in the tests have been maintained in PDA medium (Potato Dextrose Agar, Scharlau Chemie SA. SPAIN) at 4 °C. The individual strain spore suspensions have been further stored at a temperature of -20 °C in citrate buffer (0.13 M, pH 4.5) supplemented with glycerol at a final concentration of 15% (v/v).

### Preparation of a Penicillium roqueforti spore suspension from a solid substrate

Inoculation of P. *roquefortii* on a solid, husked, white rice substrate, envisaged a pre-inoculation stage on PDA medium, prepared in glass tubes (250 ml capacity) containing 100 ml of agar-containing medium sterilised in a high pressure steam autoclave at 110 °C for 20 minutes. Once sterilised, the media containing tubes are left to cool on an inclined surface such as to give a solid surface (slant) on which to perform subsequent P. *roquefortii* inoculations. Specifically, a sterile loop is used to take a minimal amount of spores from the P. *Roqueforti* maintenance culture, which is then immediately transferred sterilely onto the surface of the PDA medium, prepared as described above. The tubes containing the inoculated medium were then subsequently incubated under aerobic conditions at a temperature of 25 °C (18-30 °C) for 3-5 days, until growth and sporulation of the *Penicillium* strain had occurred. Temperatures of between 18 and 30 °C had an effect on the duration of the incubation period and on the level of sporulation.

After 3-5 days of incubation, the PDA-grown *Penicillium* biomass was resuspended in 200 ml of citrate buffer (0.13 M, pH 4.5) and the suspension thus obtained was used to inoculate 2 kg of husked, white rice, suitably sterilised and contained in 24 litre polycarbonate flasks (Nalgene Labware, USA). The rice was subjected to two 20 minute sterilisation cycles at 110 °C, without the addition of buffer or any other aqueous solution. The sterilisation process altered the water activity value from 0.57 to 0.87, and the addition of 200 ml of citrate buffer resulted in a further increase in activity to 0.95; a value comparable to that of bread, the substrate traditionally used for growing *Penicillium,* but containing gluten.

Subsequent to the addition of the *Penicillium* suspension, the flask containing the rice was subjected to gentle agitation for 1-2 minutes such as to make the distribution of the inoculum homogeneous. This was followed by the aerobic incubation stage at approx. 28 °C for 4-7 days, until an adequate level of sporulation had been achieved (10⁷ spores/ml). During incubation, the flask containing the rice was kept in a horizontal position in order to maximize the substrate surface area in contact with the air. Gentle mixing occurred at regular intervals in order to allow both the remixing of the rice with the *Penicillium,* and the aeration of the deepest parts of the substrate, during the growth phase. The level of sporulation of the mycelium was assessed after 2-4 days of incubation. To that end, a quantity, equal to about 3-5 g of substrate-mycelium was removed under sterile conditions, and 4-6 ml of citrate buffer added, followed by gentle agitation for 2 minutes. The suspension thus obtained was filtered using a Buckner funnel (fitted with a filtration septum with a cut-off of 1 mm). The filtrate spore concentration was assessed by phase contrast microscopy, using a Petroff-Hausser counting chamber. When the suspension concentration reached a value close to 10⁷ spores/ml (4-7 days), incubation was interrupted and a suitable quantity of saline solution (NaCl 25% w/v), acidified to pH 4 using lactic acid and sterilised at 110 °C for 20 minutes, varying between 1.5 and 3.5 litres, added to the culture. This was followed by gentle agitation (2-4 minutes) followed by filtration, conducted as described above. The filtrate, collected in a sterile 5 litre polycarbonate container (Nalgene Labware, USA) was then subjected to: i) evaluation of the spore load by phase contrast microscopy using a Petroff-Hausser counting chamber; ii) evaluation of spore viability by dilution followed by plating out on PDA medium; iii) evaluation of the total bacterial load on PCA (Plate Count Agar, Scharlau Chemie SA. SPAIN); iv) evaluation of the presence of any potentially contaminating moulds by microscopic evaluation of the colonies grown on PDA from the various spore suspension dilutions analysed.

The results obtained showed spore load levels varying between 10⁷ and 10⁸ spores-CFU/ml, while the total bacterial load on PCA always gave values of less than 1 CFU/ml.

The spore preparation thus obtained can be used for the preparation of "gluten free" marbled cheeses such as Roquefort, Gorgonzola, Stilton and Blue Cheese.

### EXAMPLE 2

Gorgonzola is made from whole, pasteurised cow's milk, poured into cheese vats at a temperature of approx. 30°, along with lactic ferment strains, rennin and *Penicillium* spores prepared according to example 1. Once coagulation has occurred, the curds are cut up and placed on drainers to allow the whey to run out. After a few minutes, they are arranged inside cheese moulds or bands, approx. 14/15 kg. per mould, per layer, and are left to sit to allow the whey to run off. After further resting, the moulds are then turned. After even further resting, the moulds are turned once more. The cheeses then follow the path of "purgatory" (chambers at 20/22°C with 90/95% humidity), where they are expertly salted, above, below and around the sides, and after a further 3/4 days, maturation begins. When the maturing cheese is still between the third and fourth week, perforation takes place. The cheese is punctured using large metal needles, first on one side, then round the edge and finally on the other side, thus allowing air to enter and develop the culture already primed in the curd. The air entering this way gives rise to optimal, natural conditions for development of the *Penicillium;* giving the cheese the characteristic blue/green veins which make gorgonzola unmistakable. Towards the end of the maturation process, the cheeses are removed from the wooden moulds in which they would normally stay during the two months in the refrigerator, and they are then cut in two, or into smaller fragments and each portion receives an embossed aluminium cover, the role of which is to reduce weight loss through evaporation, protect the rind against breakage or cracking, and preserve the precious organoleptic characteristics of the gorgonzola during transportation and over time.

Obviously, with the aim of satisfying contingent and specific requirements, one skilled in the art can carry out additional modifications and variations to the method for the preparation of *Penicillium* spores and the use of the latter in the food field according to the present invention, all of which are however contained within the scope of protection of the invention, as defined by the enclosed claims.

## Claims

1. A method for the preparation of gluten-free *Penicillium* spores comprising the stages of:
a) bringing *Penicillium* spores into contact with a starchy growth substrate;
b) depositing said spores on said substrate under appropriate conditions, and for such a length of time necessary to obtain the desired growth of gluten-free *Penicillium.*

2. The method according to claim 1, wherein the microorganism *Penicillium* belongs to the species *Penicillium roqueforti.*

3. The method according to claims 1 or 2, comprising a preliminary *Penicillium* preparation and maintenance stage, comprising harvesting *Penicillium* and bringing it into contact with a gluten-free growth medium.

4. The method according to claim 3, wherein the preliminary stage comprises of bringing the *Penicillium* into contact with a growth medium comprising starches, simple carbohydrates and agar.

5. The method according to claims 3 or 4, wherein the *Penicillium* is maintained under aerobic conditions for a length of time comprising between 3 and 5 days, at a temperature ranging between 18° and 30°C.

6. The method according to any claim 1 to 4, comprising a pre-inoculation stage wherein the *Penicillium* is made to grow on a sterile, starchy, gluten-free substrate, containing a source of simple carbohydrates and agar for a length of time ranging between 3 and 5 days, under aerobic conditions, and at a temperature ranging between 18°C and 30°C in order to allow growth and sporulation of the microorganism.

7. The method according to claim 6, wherein following the pre-inoculation stage, the microorganism is harvested and resuspended in a solution containing an organic acid as a source of carbon and/or capable of solubilising and providing metals which are normally used by the microorganism for performing its metabolic activities.

8. The method according to claim 7, wherein said organic acid containing solution is a buffer containing citric acid.

9. The method according to any claim 1 to 8, wherein the *Penicillium* growth substrate is a starchy substrate selected from potato and rice.

10. The method according to claim 9, wherein the growth substrate is white, husked rice that has been subjected to two cycles of sterilisation.

11. The method according to any claim 1 to 10, wherein step a) occurs by the addition of a quantity of a solution containing the spores at a concentration ranging between 10⁶ and 10⁷ spores/ml directly onto said growth substrate so that the ratio of spores/g of substrate is ranging between 10⁵ and 10⁶.

12. The method according to claim 11, wherein said solution is a solution containing an organic acid as a source of carbon and/or is capable of solubilising and providing metals which are normally used by the microorganism for performing its metabolic activities.

13. The method according to any claim 1 to 12, wherein step b) consists of an incubation stage under aerobic conditions, and at a temperature ranging between 15°C and 30°C, for 4-15 days, such as to achieve a level of sporulation ranging between 10⁶-10⁹ spores/ml.

14. The method according to claim 13, wherein, during the incubation stage, the substrate is aerated and agitated.

15. The method according to any claim 1 to 14, comprising a sporulation termination stage, achieved by means of the addition of an acidic solution.

16. The method according to claim 15, comprising a biomass separation stage, performed following the sporulation termination stage.

17. A process for the production of dairy foodstuffs, comprising an inoculation stage of said foodstuffs with *Penicillium* obtained in accordance with any claim 1 to 16.

18. Dairy foodstuffs obtainable through inoculation with *Penicillium* obtainable in accordance with any claim 1 to 16.

19. The dairy foodstuffs according to claim 18, comprising marbled cheeses such as Gorgonzola, Roquefort, Stilton and Blue Cheese.
